(19) Europäisches Patentamt
European Patent Office
Office européen des brevets

(11) Numéro de publication : **0 166 628 B1**

(12) **FASCICULE DE BREVET EUROPÉEN**

(45) Date de publication du fascicule du brevet : **19.07.89**

(51) Int. Cl.⁴ : **C 12 N 15/00**, C 12 N 1/20 // (C12N1/20, C12R1:125)

(21) Numéro de dépôt : **85400830.7**

(22) Date de dépôt : **26.04.85**

# (54) Procédé d'amplification de l'expression d'un gène déterminé chez bacillus subtilis et souches obtenues.

(30) Priorité : **27.04.84 FR 8406701**

(43) Date de publication de la demande : **02.01.86 Bulletin 86/01**

(45) Mention de la délivrance du brevet : **19.07.89 Bulletin 89/29**

(84) Etats contractants désignés : **AT BE CH DE FR GB IT LI LU NL SE**

(56) Documents cités :
**EP-A- 0 074 808**
**WO-A-84/003 81**
**GB-A- 2 068 971**
**US-A- 4 336 336**
**NATURE, vol. 283, 10 janvier 1980, page 218, MacMillan Journals Ltd.; C. GAILLARD et al.: "Excision sequences in the mitochondrial genome of yeast"**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

(72) Inventeur : **Ehrlich, Stanilas**
**4, rue Linné**
**F-75005 Paris (FR)**
Inventeur : **Janniere, Laurent**
**32, rue Mario Capra**
**F-94400 Vitry Sur Seine (FR)**
Inventeur : **Pierre, Evelyne**
**3, rue du Marineau**
**F-91580 Etrechy (FR)**
Inventeur : **Niaudet, Brigitte**
**42, avenue René Petit**
**F-75014 Paris (FR)**

(74) Mandataire : **Warcoin, Jacques et al**
**Cabinet Régimbeau 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 166 628 B1

**Description**

La présente invention concerne un procédé d'amplification de l'expression d'un gène déterminé chez les Bacillus, en particulier B. subtilis.

B. subtilis constitue actuellement un hôte de choix pour l'expression de gènes étrangers par des techniques de génie génétique car il s'agit d'une bactérie non pathogène dont les conditions de fermentation au stade industriel sont connues.

On sait depuis assez longtemps transformer des souches de B. subtilis par de l'ADN plasmidique en utilisant des plasmides capables de se répliquer.

Cependant, il existe des difficultés qui sont liées à l'instabilité de ces plasmides hybrides dans B. subtilis. On distingue au moins deux types d'instabilité.

L'instabilité ségrégationnelle correspondant à la perte totale du plasmide par la cellule hôte, et

L'instabilité structurale qui correspond à des remaniements de séquences, le plus souvent des délétions.

Ces phénomènes d'instabilité sont beaucoup plus fréquents chez B. subtilis que chez E. coli, ce qui explique en partie pourquoi cette dernière bactérie est beaucoup plus utilisée comme cellule hôte dans les techniques actuelles.

Ce problème pourrait être résolu si le clonage des gènes étrangers pouvait être effectué dans le chromosome de B. subtilis, car, dans ce cas, la stabilité est beaucoup plus grande.

Des vecteurs intégratifs ont donc été construits dans le but d'intégrer des gènes étrangers dans le chromosome de B. subtilis. Ces vecteurs se caractérisent essentiellement par l'existence de segments homologues du chromosome de B. subtilis qui assurent l'intégration.

L'intégration de ces vecteurs dans le chromosome peut s'effectuer schématiquement selon les deux processus suivants :

1. Par un événement de recombinaison impliquant un seul « crossing-over », décrit par Campbell (1962) (fig. 1). Ce mécanisme d'intégration ne peut se faire que si le vecteur est recircularisé dans la cellule réceptrice. Cette recirculation peut survenir soit à la suite d'événements de recombinaison intramoléculaire si le vecteur est doté de répétitions internes (Michel et coll., 1982), soit à la suite d'événements de recombinaison intermoléculaire avec les séquences homologues résidant dans le chromosome de la cellule réceptrice (Niaudet et coll., 1982). Le vecteur peut probablement être recircularisé par réparation de la coupure (comme démontré pour les plasmides par Michel et coll., 1982, 1983), en utilisant comme matrice des séquences homologues portées par le vecteur lui-même, s'il contient des répétitions internes, ou portées par le chromosome de la cellule réceptrice. L'intégration par un mécanisme de type Campbell crée une duplication des séquences homologues, duplication qui encadre l'ADN étranger (Haldenwang et coll., 1980 ; Niaudet et coll., 1982).

2. Par un mécanisme de double « crossing-over » (fig. 2). Le vecteur linéaire doit alors avoir deux régions d'homologie avec l'ADN chromosal de B. subtilis, situées de part et d'autre de la séquence hétérologue (Harris-Warrick et Lederberg, 1977 ; Niaudet et coll., 1982). Si les deux segments chromosomaux portés par le vecteur sont non adjacents dans le chromosome, l'intégration du vecteur par double « crossing-over » produit la délétion de la portion chromosomale située entre ces deux régions d'homologie (Niaudet et coll., 1982).

Cette intégration chromosomique dans B. subtilis ne permet pas d'obtenir une amplification génique comme cela est le cas avec les vecteurs plasmidiques autoréplicables. Or, l'amplification génique est l'une des conditions recherchées actuellement dans le domaine du génie génétique pour assurer une expression importante du gène cloné et donc la production en quantité industrielle de la protéine correspondante.

L'invention permet de remédier à ces inconvénients grâce à un procédé de préparation d'une souche de Bacillus dans le chromosome de laquelle un gène déterminé a été amplifié, c'est-à-dire est présent à plusieurs exemplaires, caractérisé en ce que :

a) on effectue l'intégration dans le chromosome de ladite souche d'au moins une séquence d'ADN dite d'intégration comportant au moins :

une séquence d'ADN dite unité amplifiable de structure :

—D—M—D—

comportant au moins ledit gène M et à chaque extrémité deux séquences identiques D dans le sens direct, ces séquences D assurant l'intégration du gène M dans un gène non essentiel de Bacillus ;

l'unité amplifiable codant, en outre, pour un gène sélectionnable ;

b) on sélectionne ensuite les souches de Bacillus obtenues par culture sur un milieu de sélection correspondant au gène sélectionnable et on prélève les souches dont le phénotype correspond à la présence d'un nombre de copies dudit gène accru par rapport à la population bactérienne avant

sélection.

L'unité amplifiable est constituée de préférence par une unité d'amplification comportant ledit gène et ses éléments d'expression ou non, l'extrémité N-terminale de cette séquence étant identique à une séquence située après l'unité d'amplification, ces deux séquences identiques étant appelées ci-après « séquences dupliquées ». Dans le cas où l'unité d'amplification ne comporte pas les éléments d'expression du gène, les séquences D devront assurer l'intégration du gène M en aval d'une séquence chromosomique d'expression capable d'assurer l'expression dudit gène. La séquence d'ADN d'intégration sera, de préférence, portée par un plasmide bactérien, bien que cela ne soit pas indispensable.

Grâce à ce procédé, on peut sélectionner des souches de B. subtilis ayant subi une amplification génique au niveau des chromosomes tant pour le marqueur de sélection que pour le gène déterminé, comme cela apparaîtra à la lecture des exemples.

De façon générale, le gène sélectionnable sera un gène de résistance à un composé chimique, en particulier un antibiotique : kanamycine, chloramphénicol, ampicilline ou tétracycline par exemple.

Dans ces conditions, la sélection de la souche est aisée puisqu'il suffit de sélectionner une souche très résistante à l'antibiotique.

Ainsi, on a pu obtenir avec une unité d'amplification comportant, outre la séquence dupliquée, le gène km et à titre de gène déterminé le gène Cm, une amplification génique de Cm en sélectionnant les souches très résistantes à Km. Cette expérience montre bien l'intérêt de la présente invention si l'on remplace Cm par un gène codant pour une protéine intéressante.

On peut obtenir une souche hyper-productrice de cette protéine, qui est stable sans qu'il soit nécessaire de maintenir une pression de sélection.

La figure 3 représente, en bas, la structure schématique d'un gène amplifié par le procédé de l'invention à partir d'une structure amplifiable en haut comportant une unité d'amplification U.A. constituée d'une séquence dupliquée D et d'une séquence M amplifiée qui comporte le gène marqueur et le gène codant pour la protéine intéressante.

Sur le plan technique, les vecteurs d'intégration sont connus dans leurs principes et leurs réalisations particulières.

Comme cela a été indiqué précédemment, il est nécessaire pour que l'intégration puisse se faire que le vecteur présente une séquence d'ADN identique à celle du chromosome de la souche de Bacillus. Dans certains cas, on utilise des vecteurs portant des fragments du chromosome de Bacillus sauvage, par exemple tout ou partie du gène thy B, ou d'un gène qui sera nommé X. Dans d'autres cas, on introduit préalablement dans le chromosome une séquence déterminée provenant, par exemple, d'un plasmide bactérien tel que pBR322, dans ce cas il suffira d'utiliser un vecteur d'intégration portant la même séquence. Cette dernière technique est particulièrement simple à mettre en œuvre.

De façon générale, le vecteur d'intégration comportera une unité d'amplification constituée d'une séquence dupliquée, d'un gène marqueur et du gène déterminé, le chromosome de Bacillus comportant, avant l'intégration, une séquence dupliquée.

La présente invention concerne également les souches de Bacillus, en particulier B. subtilis, obtenues par la mise en œuvre du procédé de l'invention, ainsi qu'un procédé de culture des souches obtenues afin de préparer le produit codé par le gène déterminé.

De façon tout à fait surprenante, on a constaté lors de la mise en œuvre des souches selon l'invention que la structure amplifiée était très stable alors qu'il est couramment admis qu'une telle structure est particulièrement instable, quelle que soit l'espèce bactérienne dans laquelle elle a été obtenue et sa localisation (chromosomale ou plasmidique). Le procédé de l'invention permet donc d'obtenir des souches stables industriellement exploitables.

D'autres caractéristiques et avantages de la présente invention apparaîtront dans les exemples de réalisation ci-après qui seront décrits en se référant aux figures sur lesquelles :

la figure 1 représente le schéma d'un simple événement de recombinaison,
la figure 2 représente le schéma d'un double événement de recombinaison,
la figure 3 représente le schéma de l'amplification génique,
la figure 4 représente la construction des souches α et A,
la figure 5 représente la structure de la souche A,
la figure 6 représente la courbe du nombre de copie du gène/résistance à la kanamycine,
la figure 7 représente la courbe du nombre de copie du gène/l'activité 4'ANT,
la figure 8 représente la construction et la structure des souches Bt, Bx et C,
la figure 9 représente la structure de la souche D,
la figure 10 représente la structure de la souche E.

Sauf indication contraire, les différentes enzymes sont mises en œuvre selon les techniques préconisées par le fournisseur.

1. Etude de l'amplification du gène de résistance à la Kanamycine dans la souche A

1.1. Construction de la souche A

Afin d'étudier l'amplification génique, on introduit dans le chromosome de B. subtilis une structure composée d'un gène de résistance à la kanamycine flanqué de deux séquences identiques, en répétition directe, dérivées de séquences de pBR322.

La souche contenant cette structure (nommée souche A) a été construite en deux étapes :

a) Insertion d'une première copie de pBR322 dans le chromosome de B. subtilis, par un événement de double crossing-over (Niaudet et coll., 1982) (obtention de la souche α) ;

b) Insertion de la deuxième copie de pBR322 et du gène Km, par un mécanisme de type Campbell (obtention de la souche A) ; les différentes étapes de cette construction sont schématisées sur la figure 4.

a) Insertion de la première copie de pBR322 (souche α)

Pour insérer la première copie de pBR322 dans le chromosome de B. subtilis, on utilise le vecteur intégratif pHV452. Il est composé de deux segments du chromosome de B. subtilis, l'un portant le gène thy B (segment appelé Thy B), l'autre ne contenant pas de fonction connue (segment appelé X) et du plasmide de E. coli pHV33Δ81 (Dagert et coll., 1984). pHV33Δ81 est composé du gène de résistance au chloramphénicol de pC194 (Iordanescu, 1975) et de pBR322 délété d'une partie du gène $Tc^R$. Grâce à ces deux segments chromosomaux, pHV452 peut s'intégrer dans le chromosome de B. subtilis et conférer à la bactérie hôte la résistance au chloramphénicol.

On a utilisé pHV452 linéarisé par l'endonucléase BglII pour transformer des cellules compétentes de la souche SB202 de B. subtilis. $10^5$ transformants $Cm^R/\mu g$ d'ADN ont été obtenus. Tous les transformants étaient $Ile^-$, ce qui indique que pHV452 linéarisé a été intégré dans le chromosome de B. subtilis à la suite d'un double événement de recombinaison entre les segments plasmidiques et chromosomaux Thy B et X (fig. 4) (Niaudet et coll., 1982). La région chromosomale originale située entre ces segments, contenant le gène ilvA, est alors délétée et remplacée par pHV33Δ81, la perte du gène ilvA conférant à la souche le phénotype $Ile^-$ (Barat et coll., 1965).

L'analyse chromosomique d'un transformant $Cm^R$ $Ile^-$ montre que celui-ci a la structure représentée sur la figure 4. La souche ainsi construite contient donc une séquence dérivée de pBR322 insérée dans les régions chromosomales Thy B et X.

b) Insertion de la deuxième copie de pBR322 et du gène $Km^R$ (souche A)

Pour construire une souche contenant une duplication des séquences de pBR322 autour du gène de résistance à la kanamycine, on a intégré dans la région pBR322 de la souche α, par un événement de recombinaison de type Campbell, un plasmide composé des séquences de pBR322 et du gène de résistance à la kanamycine.

Le plasmide utilisé, pHV457, comprend les séquences de pBR322 et les segments Sau3A I et IV et pUB110 (plasmide de Staphylococcus aureus) (Gryczan et Dubnau, 1978). Ces segments, insérés dans le site BamHI de pBR322, contiennent un gène de résistance à la kanamycine, et un site pour l'enzyme de restriction BglII (Michel et coll., 1982) (fig. 4).

Comme on a constaté qu'il était impossible de sélectionner l'intégration d'une seule copie de phV457 dans le chromosome de B. subtilis, on a introduit ce plasmide dans des cellules compétentes de la souche SB202 ($Trp^-$), en congression avec l'ADN chromosomal de la souche HVS246 ($Trp^+$). Parmi les transformants $Trp^+$ obtenus, 5 % des clones étaient résistants à la kanamycine. L'ADN chromosomal d'un transformant $Km^R$ $Trp^+$ a été extrait et analysé selon la technique d'hybridation de Southern, après restriction par BglII ou EcoRI, en utilisant pHV33Δ81 comme sonde. L'autoradiogramme obtenu montre que le transformant analysé a la structure représentée sur les figures 4 et 5. La souche A ainsi construite contient donc dans son chromosome une structure amplifiable où le gène de résistance à la kanamycine est flanqué de deux séquences homologues dérivées de pBR322. L'unité d'amplification de la souche A, telle qu'elle a été définie dans la description, est pHV457. Il est important de noter que cette souche n'a jamais été en contact direct avec la kanamycine, le phénotype de résistance à la kanamycine ayant été détecté à l'aide d'une sous-culture de cette souche.

1.2. Détection de bactéries résistantes à des concentrations sub-inhibitrices de kanamycine

Pour rechercher dans la souche A des sous-populations bactériennes résistantes à des concentrations de kanamycine supérieures à celles tolérées ar la population globale (concentrations sub-inhibitrices), on a étalé des aliquots d'une culture de la souche A sur milieu solide contenant des concentrations croissantes de kanamycine.

Les résultats montrent :

— qu'une copie du gène $Km^R$ confère la résistance à 1 μg/ml de cet antibiotique (la souche parentale SB202, qui ne contient pas ce gène, est résistante à 0,5 μg/ml de kanamycine) ;

— qu'il existe dans la souche A des bactéries résistantes à des concentrations de kanamycine supérieures à 1 μg/ml ; la fréquence de ces bactéries varie de $10^{-4}$ à $5.10^{-7}$.

1.3. Enrichissement des bactéries résistantes à des concentrations de 2 à 10 μg/ml de kanamycine

La souche A a été ensemencée en milieu liquide (la concentration initiale étant de $10^6$ cellules/ml) contenant des concentrations croissantes de kanamycine (1 à 10 μg/ml). Toutes les heures, la densité optique de ces cultures a été mesurée à 600 nM.

Les résultats de cette expérience montrent l'existence d'une sous-population spontanée résistante à 10 μg/ml de kanamycine (appelée A $Km_{10}^R$), dont la fréquence est de $5.10^{-7}$. Cette sous-population croît avec un temps de génération d'environ 20 mn. Après 8 heures de culture, elle devient majoritaire.

Par des cultures successives de la souche A (représentant 30 générations cellulaires), en milieu liquide contenant des concentrations de kanamycine échelonnées de 2 à 10 μg/ml, nous avons enrichi et purifié des souches résistantes à 2 ; 2,5 ; 3 ; 4 ; 5 ; 8 et 10 μg/ml de kanamycine.

1.4. Détection de l'amplification du gène $Km^R$ dans les souches de type A

On peut détecter une amplification génique dans un chromosome bactérien par la technique suivante : l'ADN chromosomal extrait d'une souche contenant une structure amplifiée est restreint par une endonucléase ayant un site unique de coupure dans l'unité d'amplification (U.A.). Cette restriction produit, outre les différents segments chromosomaux, un segment correspondant à l'unité d'amplification. Si le degré d'amplification est suffisant, l'analyse par électrophorèse en gel d'agarose de cette restriction permettra de visualiser une bande qui n'existe pas dans l'ADN chromosomal extrait de la souche parentale « non amplifiée » et qui correspond à l'U.A.

On a utilisé cette technique pour détecter l'amplification de pHV457 dans le chromosome des différentes souches résistantes à la kanamycine. Leur ADN chromosomal et celui de la souche A, ont été extraits puis restreints par l'endonucléase ClaI. Cette enzyme possède un seul site de coupure dans pHV457. Les produits de cette restriction ont été analysés par électrophorèse en gel d'agarose. Une bande, qui n'existe pas dans l'ADN chromosomal de la souche A, apparaît dans l'ADN des différentes souches. Son poids moléculaire est identique à celui de pHV457 et elle hybride avec des séquences de pBR322. Ces résultats suggèrent que cette bande correspond à pHV457. Sa forte intensité observée aussi bien par la technique d'électrophorèse que par celle d'hybridation indique que la structure amplifiée est composée de plusieurs copies de pHV457 disposées en répétition directes.

On a, en outre, vérifié que l'ADN total extrait des souches résistantes à la kanamycine ne contenait pas de forme extra-chromosomale, de deux façons : par électrophorèse en gel d'agarose et par hybridation selon la technique de Southern.

1.5. Détermination du nombre d'unité d'amplification/chromosome dans les souches résistantes à la kanamycine

Pour déterminer le degré d'amplification (nombre d'U.A./chromosome) dans ces souches, on a analysé par électrophorèse en gel d'agarose leurs ADN restreints par ClaI et l'ADN chromosomal de la souche A, mélangé ou non à une quantité connue de pHV457 linéarisé. En comparant par densitométrie l'intensité de la bande observée dans l'ADN de ces différentes souches à celle observée avec une quantité connue de pHV457 on a déterminé le nombre d'U.A./chromosome qu'elles contiennent. Les résultats sont représentés sur la figure 6. De 2 à 5 μg/ml de kanamycine, le nombre d'U.A./chromosome croît proportionnellement à la concentration de l'antibiotique. A 2 μg/ml de kanamycine, les bactéries contiennent 7 copies de pHV457 par chromosome, à partir de 5 μg/ml elles en contiennent 30. La taille globale de l'amplification représente environ 5 % du génome bactérien, en admettant que le poids moléculaire du chromosome est de $2,5.10^9$ D, celui de pHV457 étant de $4.10^6$ D ($30 \times 4.10^6/2,5.10^9 = 0,05$).

1.6. Accroissement du degré d'amplification

On a utilisé deux approches pour tenter d'isoler des bactéries ayant un degré d'amplification plus élevé :

a) obtention de bactéries hyper-résistantes à la kanamycine (résistantes à des concentrations supérieures à 10 μg/ml de kanamycine) ;

b) obtention de bactéries résistantes à l'amikacine (Amk).

En effet, des tests d'inhibition de croissance autour d'un disque d'antibiotique montraient que la souche A $km_{10}^R$ était plus sensible à l'amikacine qu'à la kanamycine.

Les souches résistantes de 10 à 320 μg/ml de kanamycine contiennent toutes environ 30 U.A./chromosome.

Ces résultats indiquent :

— qu'il n'existe pas de bactéries ayant des degrés d'amplification supérieurs, ou que la kanamycine ne permet pas de les sélectionner ;

— que l'hyper-résistance n'est pas liée à une augmentation du degré d'amplification du gène $Km^R$.

Bactéries résistantes à l'amikacine

En étalant une culture de la souche A $Km_{10}^R$ sur milieu solide contenant des concentrations croissantes d'amikacine, on a constaté que son efficacité d'étalement était de 50 % sur un milieu solide contenant 2,5 µg/ml d'amikacine. Pour obtenir des bactéries résistantes à des concentrations d'amikacine supérieures à 2,5 µg/ml d'amikacine , on a ensemencé la souche A $Km_{10}^R$ en milieu liquide contenant des concentrations croissantes d'amikacine, et réalisé des cycles successifs. On a ainsi isolé des souches résistantes à 4 ; 8 ; 16 ; 32 et 64 µg/ml d'amikacine (souches $AmK_4^R$ à $AmK_{64}^R$). Leur ADN a été extrait, restreint par ClaI.

L'isolation de souches résistantes à l'amikacine (jusqu'à 64 µg/ml) et l'analyse de l'ADN de ces souches montrent que ces souches contiennent environ 50 U.A./chromosome, la taille globale de l'amplification représentant 7,5 % du chromosome bactérien.

L'amikacine permet donc de sélectionner des bactéries ayant un degré d'amplification supérieur à ceux obtenus avec la kanamycine. Comme avec la kanamycine, il est possible d'isoler des souches résistantes à des concentrations croissantes d'amikacine, bien que leur degré d'amplification reste constant. On peut expliquer cette limitation du degré d'amplification en supposant que des bactéries contenant plus de 50 U.A./chromosome n'existent pas ou que l'amikacine ne permet pas de les sélectionner.

1.7. Activité de la 4'-adénosylnucléotidyl transférase (4'ANT)

La 4'-adénosylnucléotidyl transférase est une enzyme codée par le gène de résistance à la kanamycine de pUB110.

On a mesuré l'activité de cette enzyme dans les extraits bruts de la souche A et des différentes souches résistantes à des concentrations sub-inhibitrices de kanamycine.

Les résultats obtenus (fig. 7) montrent que cette activité croît proportionnellement au nombre de gènes existants dans la bactérie avec une pente voisine de 1. Ceci indique que chaque copie du gène de résistance à la kanamycine contribue de la même façon à la production de la 4'ANT. L'activité de cette enzyme a été également mesurée dans les extraits bruts des souches hyper-résistantes à la kanamycine (concentration $> 10$ µg/ml). L'activité 4'ANT qu'elles contiennent est identique à celle existant dans la souche A $Km_8^R$.

L'ensemble de ces résultats démontre que le phénotype d'hyper-résistance de ces souches n'est pas dû à une augmentation de l'activité 4'ANT ni à l'augmentation du degré d'amplification. D'autres phénomènes en sont responsables.

1.8. Stabilité de la structure amplifiée dans la A $Km_{10}^R$ (30 U.A./chromosome)

Pour étudier la stabilité de la structure amplifiée, $10^{10}$ cellules issues d'une culture fraîche de la souche A $Km_{10}^R$ ont été ensemencées en milieu liquide (100 ml) sans antibiotique. Après 3 générations, un 2$^e$ cycle de culture en milieu non sélectif a été ensemencé avec $10^8$ cellules. 5 cultures successives de ce type correspondant à 16 générations cellulaires ont été réalisées. A l'issu de chaque culture, des aliquots ont été prélevés et étalés sur milieu solide contenant 0 ou 7,5 µg/ml de kanamycine. La quantité de bactéries viables et de bactéries résistantes à 7,5 µg/ml de kanamycine existant dans ces cultures a été déterminée par numération (à chaque mesure environ 1 000 colonies ont été comptées).

Après 16 générations en milieu non sélectif, la proportion de bactéries $Km_{7,5}^R$ est supérieure à 90 % (cette valeur est la limite de fiabilité des numérations). Comme les efficacités d'étalement des souches A ayant 30, 20 ou 15 copies de pHV457 sont de 100, 25 et 0,1 % respectivement sur un milieu contenant 7,5 µg/ml de kanamycine, on peut conclure que les bactéries issues des 16 générations effectuées en milieu non sélectif ont conservé en moyenne plus de 20 U.A./chromosome.

En outre, on a calculé que la probabilité de perte du phénotype $Km_{7,5}^R$/génération est $< 5.10^{-4}$. De plus, on a extrait l'ADN chromosomal des bactéries issues de 5 générations effectuées en milieu non sélectif et mesuré leur degré d'amplification par densitométrie. Ces bactéries contenaient environ 30 U.A./chromosome dans un cas, 28 U.A./chromosome dans l'autre cas. Les analyses phénotypiques et biochimiques montrent donc une grande stabilité de la structure amplifiée.

2. Etude de l'amplification du gène de résistance à la kanamycine dans les souches B et C

La structure contenue dans la souche A permet l'amplification du gène de résistance à la kanamycine. Cette amplification est-elle due à des propriétés particulières des séquences de pBR322 ou à l'existence d'une duplication ? On a testé ces deux hypothèses en étudiant l'amplification du gène de résistance à la kanamycine :

— quand il est flanqué de deux séquences identiques autre que celles de pBR322 ;
— quand il n'est pas encadré de duplication.

2.1. Construction des souches B et C

Ces souches ont été construites en insérant dans le chromosome de B. subtilis un vecteur intégratif, soit par un mécanisme de type Campbell nécessitant un seul événement de recombinaison entre le vecteur circulaire et le chromosome, soit par un double événement de recombinaison entre le vecteur linéaire et le chromosome (Niaudet et coll., 1982).

Le vecteur intégratif utilisé est pHV458. Il est composé de pHV457 et de deux segments chromosomaux, Thy B et X (2 kb et 3,3 kb respectivement) provenant de pHV438 (Niaudet et coll., 1982) (fig. 8). Il est non réplicatif chez B. subtilis et contient le gène de résistance à la kanamycine de pUB110. Grâce à ses deux segments chromosomaux, il peut s'intégrer dans le chromosome de B. subtilis de deux façons :

a) Par un mécanisme de type Campbell dans les régions Thy B ou X créant une duplication du segment correspondant au site d'intégration, autour du gène de résistance à la kanamycine. Les deux souches ainsi construites seront appelées souches Bt et Bx (fig. 8).

b) Par un double événement de recombinaison provoquant la délétion du segment chromosomal portant le gène ilvA situé entre les régions Thy B et X, ce segment étant remplacé par pHV457 (fig. 8). La souche ainsi construite sera appelée souche C.

Comme il est impossible de sélectionner directement l'intégration d'une seule copie du gène de résistance à la kanamycine dans le chromosome de B. subtilis, pour construire ces trois souches on a transformé des cellules compétentes de la souche SB202 ($Trp^-$) par pHV458 en congression avec l'ADN chromosomal de la souche HSV246 ($Trp^+$). Parmi les transformants $Trp^+$ obtenus, on a détecté 10 % de clones $Km^R$ dont 75 % étaient $Ile^+$ et 25 % $Ile^-$.

2.1.1. Transformants $Km^R$ $Ile^-$ (souche C)

Pour vérifier que ces transformants avaient une structure de type C (fig. 8) on a extrait l'ADN chromosomal de deux transformants $Km^R$ $Ile^-$. Après restriction par EcoRI ou BglII, l'ADN a été analysé selon la technique de Southern en utilisant pHV458 comme sonde.

Les résultats obtenus après autoradiographie correspondent à ceux attendus lors de l'intégration d'un monomère de pHV458 dans la région Thy B-X du chromosome de B. subtilis par un double événement de recombinaison. Ce résultat a pu être confirmé en démontrant d'une part que ces transformants contiennent une région d'homologie avec les séquences de pB322, d'autre part qu'ils ont perdu le gène ilvA.

2.1.2. Transformants $Km^R$ $Ile^+$ (souches Bt et Bx)

Six transformants $Km^R$ $Ile^+$ ont été analysés par la technique d'hybridation de Southern. Leurs ADN chromosomaux ont été extraits puis, après restriction par EcoRI ou BglII, hybridés avec pHV458. Les résultats obtenus indiquent que quatre transformants contiennent un monomère de pHV458 intégré dans leur chromosome par un mécanisme de type Campbell, les deux autres, contenant plusieurs copies de pHV458, proviennent de l'intégration d'un polymère de pHV458 dans le chromosome de B. subtilis.

Les résultats obtenus ci-dessus ne permettaient pas de connaître le site d'intégration de pHV458 dans le chromosome de la souche SB202 (Thy B ou X). Pour le déterminer, on a restreint l'ADN chromosomal des transformants contenant un monomère de pHV458, ainsi que celui de la souche C, par l'endonucléase BamHI. Cette enzyme reconnaît un site unique dans pHV458 (fig. 8). Ces ADN restreints ont ensuite été hybridés avec pHV458 selon la technique de Southern. L'ADN de la souche C présentait une bande d'hybridation correspondant à un segment d'environ 6 kb. Or, la restriction de l'ADN chromosomal de la souche D par BamHI produit deux segments pouvant être hybridés avec pHV458 (fig. 8) dont la taille dépend de la position des prochains sites BamHI dans l'ADN chromosomal. Il s'en suit que le segment contenant le gène thyB doit avoir une taille supérieure à 2,4 kb, tandis que celui correspondant à X doit avoir une taille supérieure à 9 kb. Le segment de 6 kb détecté par hybridation correspond donc à celui contenant le gène thyB, l'autre contenant le segment X, dont la taille est supérieure à 9 kb, n'a pas été détecté, probablement parce qu'il était trop grand pour être efficacement transféré sur le filtre de nitrocellulose. Les ADN de 2 des 4 transformants contenant un monomère de pHV458 présentent une bande analogue à celle observée dans la souche C. L'analyse des structures attendues des souches Bt et Bx (fig. 8) montre que seuls les ADN contenant une structure de type Bt peuvent générer cette bande.

Les deux transformants ayant une bande de 6 kb commune avec la souche C proviennent donc de l'intégration de pHV458 par un mécanisme de type Campbell dans la région chromosomale ThyB. La souche ainsi construite contient donc une duplication des segments ThyB encadrant le gène de résistance à la kanamycine (souche Bt). Les 2 autres transformants proviennent de l'intégration de pHV458 dans la région chromosomale X. Les souches contiennent une duplication des segments X

encadrant le gène de résistance à la kanamycine (souche Bx) (fig. 8). Comme pour les souches A et C, ces deux souches (Bt et Bx) n'ont jamais été en contact direct avec la kanamycine.

2.2. Amplification dans les souches B

2.2.1. Détection de bactéries de type B résistantes à des concentrations sub-inhibitrices de kanamycine

Des aliquots des cultures des souches Bt et Bx ont été étalés sur des milieux solides contenant des concentrations croissantes de kanamycine pour déterminer leurs efficacités d'étalement sur différentes concentrations. Les résultats mettent en évidence :

a) que la souche Bx se comporte comme la souche A, elle résiste à 1 µg/ml de kanamycine et contient des bactéries capables de résister à des concentrations de kanamycine sub-inhibitrices (2 à 8 µg/ml) ; la fréquence de ces bactéries varie de $10^{-4}$ à $10^{-6}$ ;

b) la souche Bt est plus résistante à la kanamycine que les souches A et Bx, puisque son efficacité d'étalement sur 2 µg/ml de kanamycine est de $5.10^{-1}$ (au lieu de $10^{-4}$), elle contient également des bactéries capables de croître sur 4 ; 8 et 16 µg/ml de kanamycine. Il est possible que la résistance élevée à la kanamycine de cette souche provienne d'une transcription accrue du gène $Km^R$ due à un promoteur d'origine chromosomale.

2.2.2. Enrichissement de bactéries résistantes à 10 µg/ml de kanamycine

Pour enrichir les bactéries résistantes à 10 µg/ml de kanamycine, les souches Bt et Bx ont été cultivées en milieu liquide contenant 10 µg/ml de kanamycine comme pour la souche A.

Comme pour la souche A, on a pu mettre en évidence dans une culture de la souche Bx en milieu sélectif un enrichissement d'une sous-population résistante à la kanamycine. En milieu non sélectif cette sous-population n'est pas enrichie.

2.2.3. Obtention de bactéries de type Bx, hyper-résistantes à la kanamycine

A l'aide de cycles successifs en milieu liquide contenant des concentrations croissantes de kanamycine, des souches issues de la souche Bx, résistantes de 10 µg/ml à 0,1 g/ml de kanamycine, ont été obtenues.

2.2.4. Détermination du degré d'amplification des souches de type B

Le nombre de copies de pHV458 contenues dans les souches Bx résistantes de 10 µg à 20 mg/ml de kanamycine a été déterminé par densitométrie. Ces souches contiennent toutes environ 20 U.A./chromosome.

Comme pour la souche A, l'augmentation de la résistance à la kanamycine des souches de type Bx n'est pas associée à l'augmentation de leur degré d'amplification. L'hyper-résistance de ces souches est donc due à d'autres phénomènes que l'amplification génique.

Par densitométrie, nous avons constaté que la souche de type Bt résistante à 10 µg/ml de kanamycine contenait environ 5 U.A./chromosome. On suppose que l'utilisation de la kanamycine comme agent sélectif ne peut permettre d'isoler des souches de type Bt ayant un degré d'amplification supérieur puisque les souches de type A et Bx résistantes à 10 µg/ml de kanamycine contiennent un degré d'amplification maximal.

On peut probablement expliquer le faible degré d'amplification de la souche Bt $Km^R{}_{10}$ en supposant que le promoteur d'origine chromosomal provoquant la sur-expression de ce gène est contenu dans l'unité d'amplification. Sachant que le gène de résistance à la kanamycine est orienté dans le sens du petit segment BglII-BamHI de pHV457 (J. E. Davis, communication personnelle) et connaissant les structures des souches A, Bt et Bx, ainsi que leur degré de résistance à la kanamycine, on peut conclure que ce promoteur d'origine chromosomal est situé dans le segment Thy B.

2.2.5. Stabilité de la structure amplifiée dans la souche $Bx^R{}_{10}$ (20 U.A./chromosome)

La stabilité de la structure amplifiée a été étudiée dans la souche Bx $Km^R{}_{10}$ de la même façon que pour la souche A $Km^R{}_{10}$.

La stabilité de la structure amplifiée dans la souche Bx $Km^R{}_{10}$ (20 U.A./chromosome) est similaire à celle observée dans la souche A $Km^R{}_{10}$. La probabilité pour qu'une cellule $Km^R{}_{7,5}$ devienne $Km^s{}_{7,5}$ par génération est également $< 6.10^{-4}$.

La stabilité de la structure amplifiée dans la souche Bx étant identique à celle existant dans la souche A $Km^R{}_{10}$, on peut conclure que la région chromosomale délétée dans la souche A, mais présente dans la souche Bx, ne contient pas de fonctions particulières pouvant modifier la stabilité de la structure amplifiée.

2.3. Souche C

2.3.1. Obtention de souches de type C hyper-résistantes à la kanamycine et détermination de leur degré d'amplification

A partir de la souche C, résistantes à 1 µg/ml de kanamycine, on a obtenu des souches résistantes à 10-20 ... 320 µg/ml de kanamycine. Aucune amplification du gène de résistance à la kanamycine n'a été détectée dans ces souches. Ces résultats suggèrent que dans la souche C il n'y a pas de sous-population contenant une structure amplifiée. Il apparaît donc qu'une duplication autour de ce gène est indispensable pour qu'il puisse être amplifié. L'hyper-résistance des souches de type C obtenue ci-dessus serait due à une modification de la sensibilité de la souche vis-à-vis de la kanamycine.

3. Etude de l'amplification du gène de résistance au chloramphénicol (Cm) dans les souches α et D

Les études effectuées avec les souches de type A, Bx, t et C montrent que le gène de résistance à la kanamycine peut être amplifié uniquement s'il est inséré entre deux séquences identiques. Pour généraliser ces observations et montrer que l'amplification observée n'est pas due à des propriétés particulières du fragment d'ADN contenant le gène de résistance à la kanamycine, on a étudié l'amplification du gène de résistance au chloramphénicol lorsqu'il est encadré ou non de séquences identiques.

3.1. Constructions

3.1.1. Souche α

La construction de la souche α est décrite dans le paragraphe 1.1.a). Cette souche contient dans son chromosome des séquences dérivées de pBR322, portant le gène de résistance au chloramphénicol de pC194 (pHV33Δ81), non encadrées de séquences identiques (fig. 4).

3.1.2. Souche D

Dans le chromosome de la souche D existe une duplication identique à celle contenue dans la souche A (duplication de séquences dérivées de pBR322) encadrant le gène de résistance au chloramphénicol de pC194. Pour construire cette souche, on a transformé des cellules compétentes de la souche C par un plasmide non réplicatif chez B. substilis, pHV33Δ81 (Dagert et coll., 1983).

La préparation du plasmide pHV33Δ81 était fortement polymérisée, afin d'éviter l'intégration de polymères de pHV33Δ81 dans le chromosome de la souche C, on a transformé des cellules compétentes de cette souche par l'ADN de la préparation plasmidique de pHV33Δ81 préalablement linéarisé par PstI. Ainsi linéarisé, ce plasmide conserve une partie de son pouvoir transformant car la coupure qu'il porte peut être réparée par des interactions entre le plasmide linéarisé et les séquences homologues existant dans la cellule réceptrice (Contente et Dubnau, 1979). $2.10^3$ transformants $Cm^R$/µg d'ADN ont été obtenus. L'ADN total extrait d'un transformant, coupé par EcoRI et BglII a été hybridé avec pHV457. Sa structure correspondait à celle de la souche D recherchée (fig. 9).

3.2. Amplification du gène de résistance au chloramphénicol

3.2.1. Souche D

A partir de cultures successives de la souche D (résistante à 5 µg/ml de chloramphénicol) effectuées en milieu liquide contenant des concentrations croissantes de chloramphénicol, on a obtenu des souches résistantes de 10 à 50 µg/ml de chloramphénicol.

A partir d'une culture de la souche D, il est possible d'isoler des souches ayant des degrés d'amplification différents et contenant 2,5 et 7 U.A./chromosome. L'amplification du gène de résistance à la kanamycine observée dans les souches A, Bx et Bt n'est donc pas due à des propriétés particulières des séquences portant le gène $Km^R$.

Dans les conditions expérimentales, il n'a donc pas été possible d'isoler des souches contenant plus de 7 U.A./chromosome. Deux hypothèses peuvent expliquer ce résultat : soit le chloramphénicol ne permet pas de les sélectionner, soit elles n'existent pas.

3.2.2. Souche α

La souche α résiste à 5 µg/ml de chloramphénicol. A partir de cette souche il n'a pas été possible d'obtenir de bactéries résistantes à des concentrations supérieures à 5 µg/ml de chloramphénicol. L'ADN total de la souche résistante à 5 µg/ml de chloramphénicol a été extrait et analysé par densitométrie. Aucune structure amplifiée n'a été détectée.

Ces études effectuées sur les souches D et α démontrent que le gène de résistance au chloramphénicol peut être amplifié s'il est inséré entre deux séquences identiques.

4. Co-amplification de deux gènes

Pour démontrer qu'il est possible de co-amplifier deux gènes, on a construit la souche E. Dans son chromosome ont été insérés pHV33Δ81, qui contient le gène de résistance au chloramphénicol, et pHV458, qui contient le gène de résistance à la kanamycine. Ces deux plasmides encadrés de deux séquences X constituent l'U.A. n° 2 (fig. 10). Avec cette structure, l'amplification de pHV458 entraîne obligatoirement celle de pHV33Δ81. La souche E contient également une duplication des séquences de pBR322 encadrant le gène de résistance au chloramphénicol, pHV33Δ81 constituant l'U.A. n° 1 (fig. 10). L'amplification de pHV33Δ81 peut être indépendante de celle de pHV458.

4.1. Construction de la souche E

Des cellules compétentes de la souche Bx ont été transformées pour la résistance au chloramphénicol par le plasmide pHV33Δ81 préalablement linéarisé par PstI (voir paragraphe 3.1.2). $10^2$ transformants/μg d'ADN résistants à 3 μg/ml de chloramphénicol ont été obtenus. Un clone a été choisi, dont on suppose qu'il contient la structure indiquée sur la figure 10.

4.2. Amplification de pHV458 et pHV33Δ81

Les cellules de la souche E ont été ensemencées en milieu liquide contenant 10 μg/ml de kanamycine ou des concentrations croissantes de chloramphénicol (de 0 à 40 μg/ml). Après 18 heures d'incubation, la proportion de cellules résistantes à 10 μg/ml de kanamycine et 25 μg/ml de chloramphénicol a été déterminée par étalement sur milieu solide.

A l'issue d'une culture de la souche E en milieu non sélectif, la proportion de cellules résistantes à 10 μg/ml de kanamycine est de $2,5.10^{-5}$ (valeur proche de celles déterminées pour les souches A et Bx). La proportion de cellules résistantes à 25 μg/ml de chloramphénicol est de $3,3.10^{-5}$. Comme attendu, les bactéries résistantes à 10 μg/ml de kanamycine ont été enrichies pendant la culture en milieu liquide contenant 10 μg/ml de kanamycine (efficacité d'étalement de 1 sur milieu solide $Km_{10}$). Cet enrichissement est accompagné d'une augmentation de la quantité de bactéries résistantes à 25 μg/ml de chloramphénicol, efficacité d'étalement de 0,1 sur milieu solide $Cm_{25}$. La non induction du gène de résistance au chloramphénicol pendant la culture en présence de 10 μg/ml de kanamycine doit être la cause de la moins bonne efficacité d'étalement sur $Cm_{25}$ que sur $Km_{10}$(1/10).

Ces résultats montrent que l'amplification d'un gène (gène $Km^R$) entraîne celle d'un autre gène (gène $Cm^R$) présent dans la même unité d'amplification.

Pour les cultures effectuées en faibles concentrations de chloramphénicol (jusqu'à 15 μg/ml), la co-amplification des gènes de résistance au chloramphénicol et à la kanamycine semble faible. Ceci peut être dû soit à une amplification indépendante de pHV33Δ81 (il fait partie de l'U.A. n° 1 (fig. 10), soit à une co-amplification insuffisante du gène de résistance à la kanamycine (le degré d'amplification serait trop faible pour conférer la résistance à 10 μg/ml de kanamycine). Il est intéressant de noter qu'aux concentrations de chloramphénicol supérieures à 15 μg/ml, l'enrichissement des bactéries résistantes au chloramphénicol est accompagné de celui de bactéries résistantes à 10 μg/ml de kanamycine (la proportion de cellules $Km^R_{10}$ par rapport aux cellules $Cm^R_{25}$ croît de 50 % à 90 %). Ceci indique que l'amplification de l'U.A. n° 2 se fait préférentiellement à celle de l'U.A. n° 1 seule.

Les caractéristiques et les origines des souches et des plasmides utilisés dans les exemples de réalisation seront résumées dans les tableaux ci-après.

(Voir Tableau page 11)

| SOUCHES BACTERIENNES | MARQUEURS GENETIQUES | ORIGINE |
|---|---|---|
| *E. coli* | | |
| HVC45 | *thrA1 leu-6 thi-1 lacY1 tonA21 supE44 hsdR rpsL* | R. Davis |
| *B. subtilis* | | |
| SB202 | *trpC2 tyrA1 aroB2 hisH2* | P. Schaeffer |
| α | (a) *trpC2 tyrA1 aroB2 hisH2* *ins* [pHV452] *del* [ilvA2] | ce travail |
| A | (a) *tyrA1 aroB2 hisH2 ins* [pHV452] *del* [ilvA2] *ins* [pHV457] *dup* [pBR322Δ81] | ce travail |
| BT | (a) *tyrA1 aroB2 hisH2* *ins* [pHV458, ThyB] *dup* [ThyB] *dup* [X] | ce travail |
| BX | (a) *tyrA1 aroB2 hisH2* *ins* [pHV458, X] *dup* [X] *dup* [ThyB] | ce travail |
| C | (a) *tyrA1 aroB2 hisH2* *ins* [pHV458] *del* [ilvA2] | ce travail |
| D | (a) *tyrA1 aroB2 hisH2* *ins* [pHV458] *del* [ilvA2] *ins* [pHV33Δ81] *dup* [pBR322Δ81] | ce travail |
| E | (a) *tyrA1 aroB2 hisH2* *ins* [pHV458, X] *dup* [X] *dup* [ThyB] *ins* [pHV33Δ81] *dup* [pBR322Δ81] | ce travail |

(a) modifications génétiques dues à des insertions de plasmides dans le chromosome de B. subtilis :

1) ins suivi d'un nom de plasmide entre crochets signifie que le plasmide est inséré dans le chromosome

2) del ou dup suivi d'un nom de gène, de séquence ou d'un nom de plasmide signifie qu'après l'insertion du plasmide il y a eu délétion ou duplication respectivement de ce gène, séquence ou plasmide.

3) Quand un plasmide peut être inséré dans plusieurs endroits, son site d'intégration est indiqué derrière le nom du plasmide.

| PLASMIDES | CONSTRUCTION | REFERENCE |
|---|---|---|
| pBR322 | vecteur de clonage | Bolivar et col., 1977 |
| pC194 | isolat naturel | Iordanescu, 1975 |
| pUB110 | isolat naturel | Gryczan et col., 1978 |
| pHV32 | revertant $Tc^R$ obtenu *in vivo* de pHV33 | Primrose et Ehrlich, 1981 |
| pHV33 | hybride entre pC194 et pBR322 | " " |
| pHV33Δ81 | pHV33 coupé par *Bam*HI érodé par BAL31 | Dagert et col., 1984 |

(Suite)

| PLASMIDES | CONSTRUCTION | REFERENCE |
|---|---|---|
| pHV438 | hybride entre pHV32 et les segments ThyB et X du chromosome de *B. subtilis* | Niaudet et col., 1982 |
| pHV452 | hybride entre pHV33Δ81 et les séquences du chromosome de *B. subtilis* ThyB et X | ce travail |
| pHV457 | segments *Sau*3A I et IV de pUB110 inséré dans site *Bam*H1 de pBR322 | ce travail |
| pHV458 | hybride entre pHV457 et les segments du chromosome de *B. subtilis* ThyB et X | ce travail |

Références


Barat M., Anagnostopoulos C. et Schneider A.M. (1965) J. Bacteriol. 90 : 351-369

Campbell A. (1962) Advances Genet. 11 : 101-145

Contente S. et Dubnau D. (1979) Plasmid 2 : 555-571

Dagert M., Jones I.M., Goze A., Romac S., Niaudet B. et Ehrlich S.D. (1984) EMBO J. 3 : 81-86

Gryczan T.J. et Dubnau D. (1978) Proc. Natl. Acad. Sci. U.S.A. 75 : 1428-1432

Haldenwang S. Banner C.D.B., Ollington J.F., Losick R., Hoch J.A., O'Connor M.B. et Sonensheim B.L. (1980) J. Bacteriol. 142 : 90-98

Harris-Warwick R.M. et Lederberg J. (1977) J. Bacteriol. 133 : 1246-1253

Iordanescu S. (1975) J. Bacteriol. 124 : 597-601

Michel B., Niaudet B. et Ehrlich S.D. (1982) EMBO J. 1 : 1565-1571

Michel B., Niaudet B. et Ehrlich S.D. (1983) Plasmid 10 : 1-10

Niaudet B., Goze A. et Ehrlich S.D. (1982) Gene 19 : 277-284


## Revendications

1. Procédé de préparation d'une souche de Bacillus dans le chromosome de laquelle un gène déterminé M a été amplifié, c'est-à-dire est présent à plusieurs exemplaires, caractérisé en ce que :

a) on effectue l'intégration dans le chromosome de ladite souche d'au moins une séquence d'ADN dite d'intégration comportant au moins :

une séquence d'ADN dite unité amplifiable de structure :

—D—M—D—

comportant au moins ledit gène M et à chaque extrémité deux séquences identiques D dans le sens direct, ces séquences D assurant l'intégration du gène M dans un gène non essentiel de Bacillus ;

l'unité amplifiable codant, en outre, pour un gène sélectionnable ;

b) on sélectionne ensuite les souches de Bacillus obtenues par culture sur un milieu de sélection correspondant au gène sélectionnable et on prélève les souches dont le phénotype correspond à la présence d'un nombre de copies dudit gène accru par rapport à la population bactérienne avant sélection.

2. Procédé selon la revendication 1, caractérisé en ce que la séquence d'ADN dite d'intégration est portée par un plasmide bactérien.

3. Procédé selon l'une des revendications 1 et 2, caractérisé en ce que l'unité amplifiable comporte les éléments d'expression du gène M.

4. Procédé selon l'une des revendications 1 et 2, caractérisé en ce que les séquences D assurent l'intégration du gène M en aval d'une séquence chromosomique d'expression capable d'assurer l'expression dudit gène.

5. Procédé selon l'une des revendications 1 à 4, caractérisé en ce que le marqueur de sélection est

un gène de résistance à un composé chimique et en ce que à l'étape b) on sélectionne les souches présentant la résistance la plus élevée à ce composé chimique.

6. Procédé selon la revendication 5, caractérisé en ce que le composé chimique est un antibiotique.

7. Procédé selon la revendication 6, caractérisé en ce que le marqueur de sélection est le gène Km ou Cm

8. Procédé selon l'une des revendications 1 à 7, caractérisé en ce que les séquences identiques dites séquences dupliquées proviennent d'un plasmide bactérien.

9. Procédé selon la revendication 8, caractérisé en ce que les séquences dupliquées proviennent de pBR322.

10. Procédé selon l'une des revendications 1 à 8, caractérisé en ce que les séquences dupliquées sont constituées par tout ou partie d'une séquence d'ADN du chromosome de B. subtilis sauvage.

11. Procédé selon l'une des revendications 8 à 10, caractérisé en ce que les séquences dupliquées sont constituées par tout ou partie du gène thy B.

12. Procédé selon l'une des revendications 1 à 11, caractérisé en ce que dans l'étape a) le vecteur plasmidique d'intégration comporte au moins une unité d'amplification constituée d'une séquence dupliquée, d'un gène marqueur et du gène déterminé, le chromosome du Bacillus comportant, avant l'intégration, une séquence dupliquée.

13. Procédé selon la revendication 12, caractérisé en ce que la séquence dupliquée présente dans le chromosome de Bacillus a été introduite par intégration à l'aide d'un vecteur plasmidique d'intégration.

14. Souche de Bacillus obtenue par la mise en œuvre du procédé selon l'une des revendications 1 à 13.

15. Souche selon la revendication 14, caractérisée en ce qu'il s'agit de B. subtilis.

16. Procédé de préparation d'une protéine déterminée, caractérisé en ce qu'on cultive une souche selon l'une des revendications 14 et 15 dans laquelle le gène déterminé code pour ladite protéine, dans un milieu de culture, et en ce que l'on sépare ensuite la protéine exprimée.

**Claims**

1. Method for preparing a Bacillus strain in the chromosome of which a specified gene M has been amplified, that is to say is present in several copies, characterized in that :

a) the integration is performed, in the chromosome of the said strain, of at least one DNA sequence, referred to as an integration sequence, containing at least :

one DNA sequence, referred to as an amplifiable unit, of structure :

—D—M—D—

containing at least the said gene M and, at each end, two identical sequences D in the direct orientation, these sequences D providing for the integration of the gene M in a non-essential gene of Bacillus ;

the amplifiable unit coding, in addition, for a selectable gene ;

b) the Bacillus strais obtained by culturing on a selection medium corresponding to the selectable gene are selected, and the strains of which the phenotype corresponds to the presence of an increased copy number of the said gene, compared with the bacterial population before selection, are withdrawn.

2. Method according to Claim 1, characterized in that the DNA sequence referred to as an integration sequence is carried by a bacterial plasmid.

3. Method according to one of Claims 1 and 2, characterized in that the amplifiable unit contains the elements for expression of the gene M.

4. Method according to one of Claims 1 and 2, characterized in that the sequences D provide for the integration of the gene M downstream of a chromosomal expression sequence capable of providing for the expression of the said gene.

5. Method according to one of Claims 1 to 4, characterized in that the selection marker is a gene for resistance to a chemical compound and in that, in the stage b), the strains showing the highest resistance to this chemical compound are selected.

6. Method according to Claim 5, characterized in that the chemical compound is an antibiotic.

7. Method according to Claim 6, characterized in that the selection marker is the Km or Cm gene.

8. Method according to one of Claims 1 to 7, characterized in that the identical sequences, referred to as duplicated sequences, originate from a bacterial plasmid.

9. Method according to Claim 8, characterized in that the duplicated sequences originate from pB322.

10. Method according to one of Claims 1 to 8, characterized in that the duplicated sequences consist of all or part of a DNA sequence of the chromosome of wild-type B. subtilis.

11. Method according to one of Claims 8 to 10, characterized in that the duplicated sequences consist of all or part of the thy B gene.

12. Method according to one of Claims 1 to 11, characterized in that, in the stage a), the plasmid integration vector contains at least: one amplification unit consisting of a duplicated sequence, of a

marker gene and of the specified gene, the Bacillus chromosome containing, before the integration, a duplicated sequence.

13. Method according to Claim 12, characterized in that the duplicated sequence present in the Bacillus chromosome has been introduced by integration using a plasmid integration vector.

14. Bacillus strain obtained by carrying out the method according to one of Claims 1 to 13.

15. Strain according to Claim 14, characterized in that it is B. subtilis.

16. Method for preparing a specified protein, characterized in that a strain according to one of Claims 14 and 15 in which the specified gene codes for the said protein is cultured in a culture medium, and in that the protein expressed is then separated.

**Patentansprüche**

1. Verfahren zur Herstellung eines Bacillus-Stammes, in dessen Chromosom ein als M bezeichnetes Gen vermehrt wurde, d. h. in mehreren Exemplaren vorhanden ist, dadurch gekennzeichnet, daß :

a) man die Integration in das Chromosom des Stammes mittels einer ADN-Sequenz, die als zur Integration bezeichnet wird, bewirkt, die mindestens aufweist :

eine als vermehrbare Einheit bezeichnete ADN-Sequenz mit der Struktur :

—D—M—D—

die zumindest das genante Gen M und an jedem Ende zwei identische Sequenzen D in direktem Sinne aufweist, wobei diese Sequenzen D die Integration des Gens M in ein nicht essentielles Gen des Bacillus sicherstellen ;

eine vermehrbare Einheit, darüberhinaus codierend für ein selektierbares Gen ;

b) man anschließend die Stämme des Bacillus erhalten durch Kultur auf einem Selektionsmedium entsprechend dem selektierbaren Gen selektiert, und daß man die Stämme, deren Phänotyp der Anwesenheit einer Anzahl von Kopien dieses Gens, das bezogen auf die Bakterienpopulation vor der Selektion vermehrt ist, entspricht, entnimmt.

2. Verfahren nach Anspruch 1, dadurch gekennzeichnet, daß die zur Integration bezeichnete ADN-Sequenz von einem Bakterienplasmid getragen wird.

3. Verfahren nach einem der Ansprüche 1 und 2, dadurch gekennzeichnet, daß die vermehrbare Einheit die Expressionselemente des Gens M umfaßt.

4. Verfahren nach einem der Ansprüche 1 und 2, dadurch gekennzeichnet, daß die Sequenzen D die Integration des Gens M sicherstellen, folgend einer Expressions-Chromosomensequenz, die geeignet ist, die Expression dieses Gens sicherzustellen.

5. Verfahren nach einem der Ansprüche 1 bis 4, dadurch gekennzeichnet, daß der Selektionsmarkierer ein Gen mit Widerstandsfähigkeit gegenüber einer chemischen Verbindung ist, und daß in der Stufe b) die Stämme selektiert werden, die die größte Resistenz gegenüber dieser chemischen Verbindung aufweisen.

6. Verfahren nach Anspruch 5, dadurch gekennzeichnet, daß die chemische Verbindung ein Antibiotikum ist.

7. Verfahren nach Anspruch 6, dadurch gekennzeichnet, daß der Selektionsmarkierer das Gen Km oder Cm ist.

8. Verfahren nach einem der Ansprüche 1-7, dadurch gekennzeichnet, daß die identischen Sequenzen, die als verdoppelte Sequenzen bezeichnet werden, von einem bakteriellen Plasmid stammen.

9. Verfahren nach Anspruch 8, dadurch gekennzeichnet, daß die verdoppelten Sequenzen von pBR322 stammen.

10. Verfahren nach einem der Ansprüche 1-8, dadurch gekennzeichnet, daß die verdoppelten Sequenzen vollständig oder teilweise aus einer Sequenz von ADN des Chromosoms von unkultiviertem B. subtilis gebildet werden.

11. Verfahren nach einem der Ansprüche 8-10, dadurch gekennzeichnet, daß die verdoppelten Sequenzen vollständig oder teilsweise vom Gen thy B gebildet werden.

12. Verfahren nach einem der Ansprüche 1-11, dadurch gekennzeichnet, daß in der Stufe a) der Plasmid-integrationsvektor zumindest umfaßt eine Vermehrungseinheit, die aus einer Verdoppelungssequenz, einem Markierergen und dem bestimmten Gen gebildet wird, wobei das Chromosom des Bacillus vor der Integration eine verdoppelte Sequenz umfaßt.

13. Verfahren nach Anspruch 12, dadurch gekennzeichnet, daß die in dem Chromosom des Bacillus vorhandene verdoppelte Sequenz in dem Chromosom des Bacillus eingeführt wurde durch Integration mittels eines Plasmid-Integrationsvektors.

14. Bacillusstamm, erhalten durch Durchführung des Verfahrens nach einem der Ansprüche 1-13.

15. Stamm nach Anspruch 14, dadurch gekennzeichnet, daß es sich um B. subtilis handelt.

16. Verfahren zur Herstellung eines bestimmten Proteins, dadurch gekennzeichnet, daß man einen Stamm nach einem der Ansprüche 14 und 15, worin das bestimmte Gen für das Protein codiert, in einem Kulturmedium kultiviert und daß man anschließend das exprimierte Protein abtrennt.

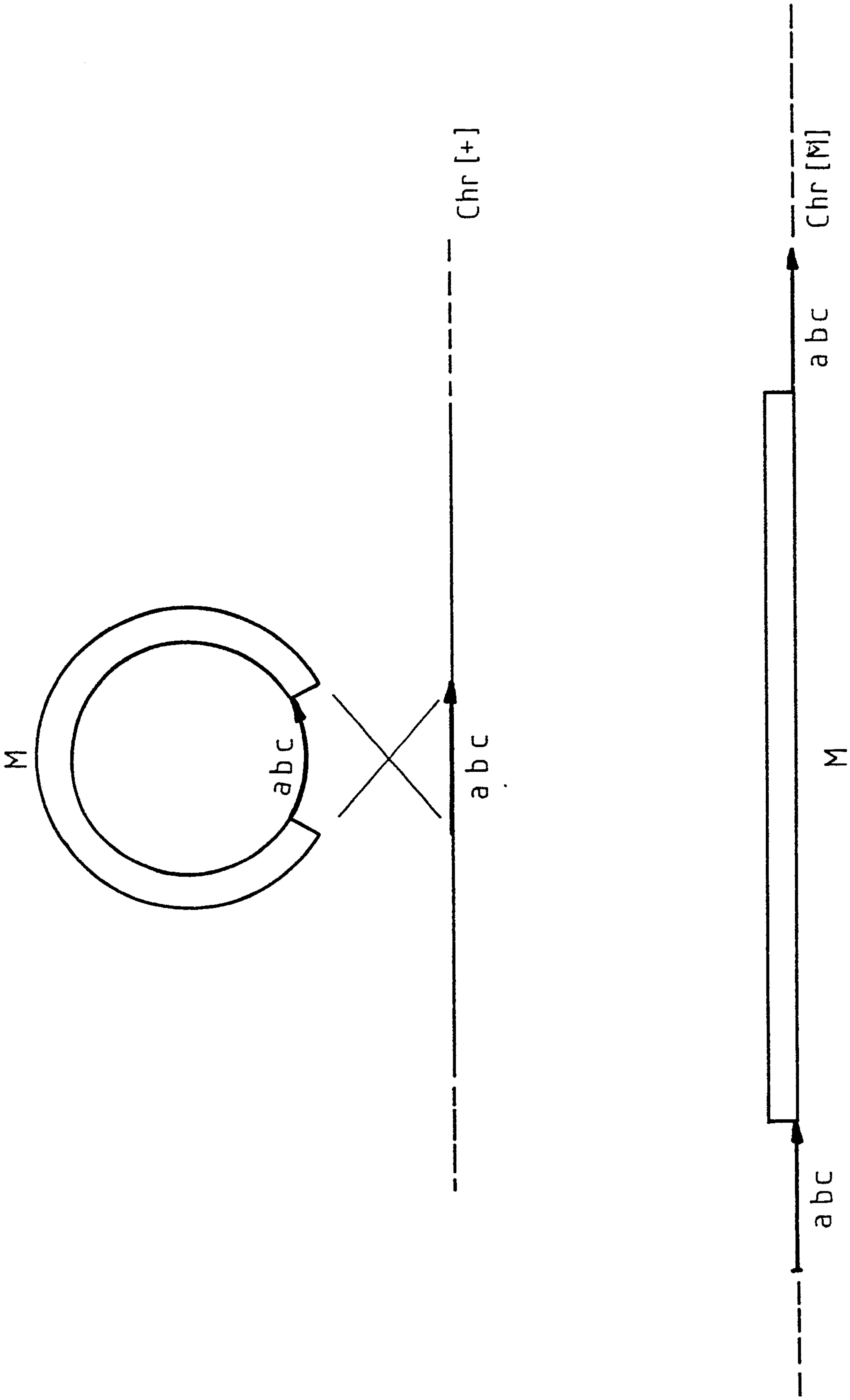


FIG-1

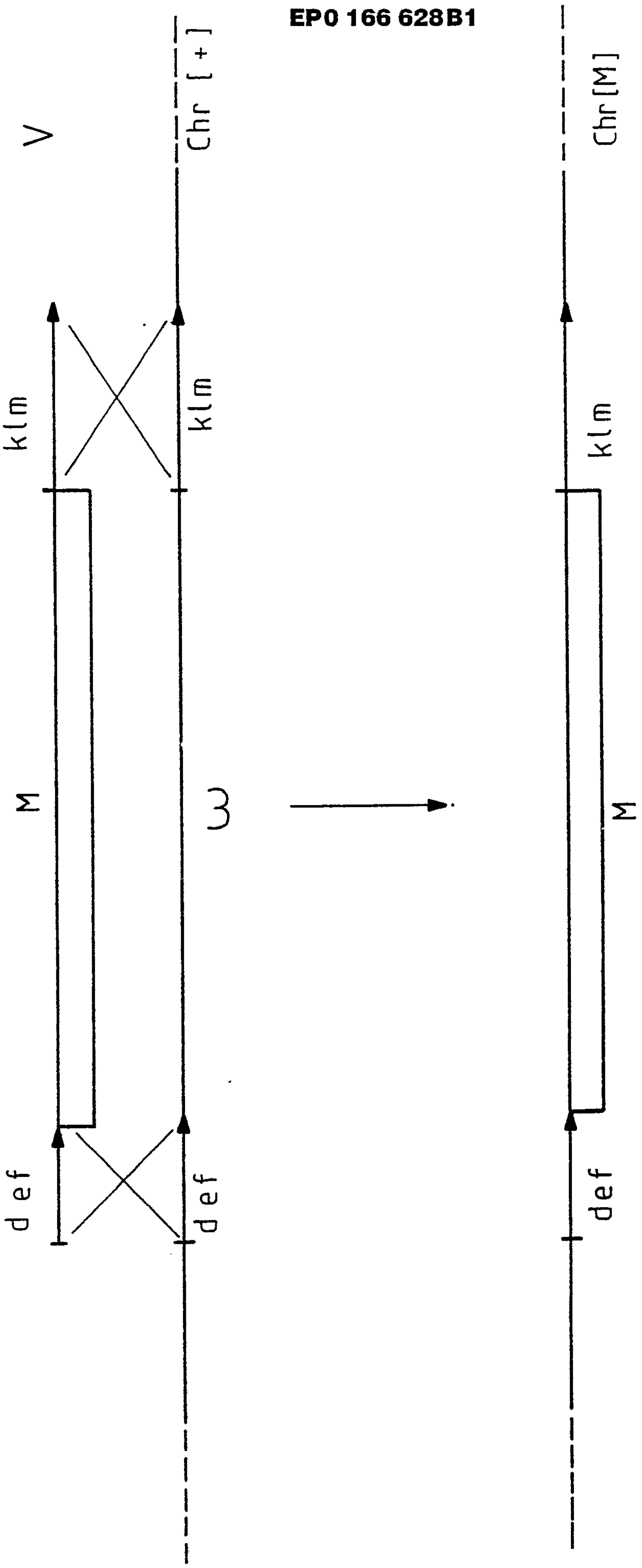


FIG-2

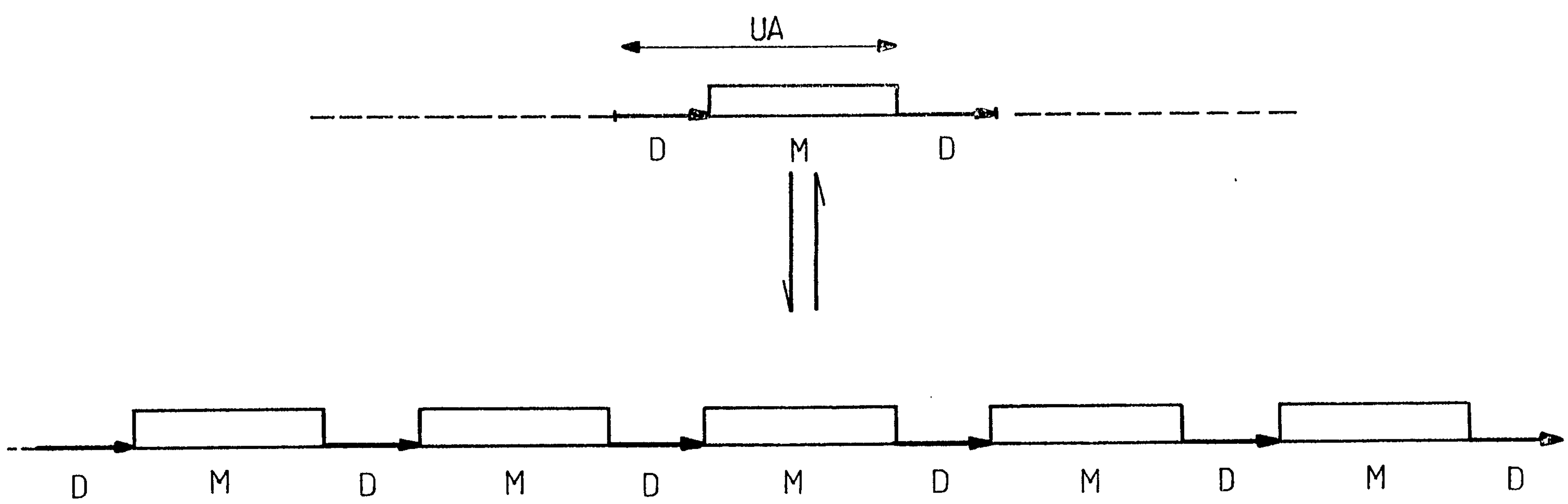
UA
D
M
D
D
M
D
M
D
M
D
M
D
M
D

FIG-3

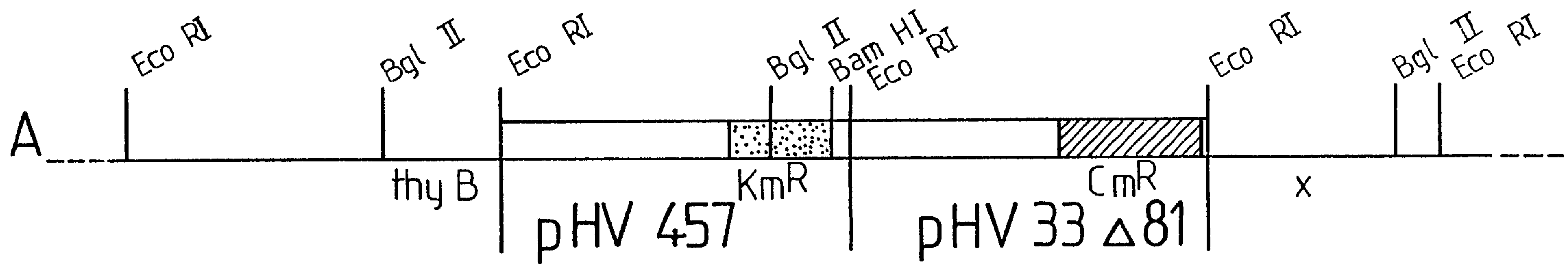
A
Eco RI
Bgl II
Eco RI
Bgl II
Bam HI
Eco RI
Eco RI
Bgl II
Eco RI
thy B
KmR
CmR
x
pHV 457
pHV 33 Δ81

FIG-5

FIG-4

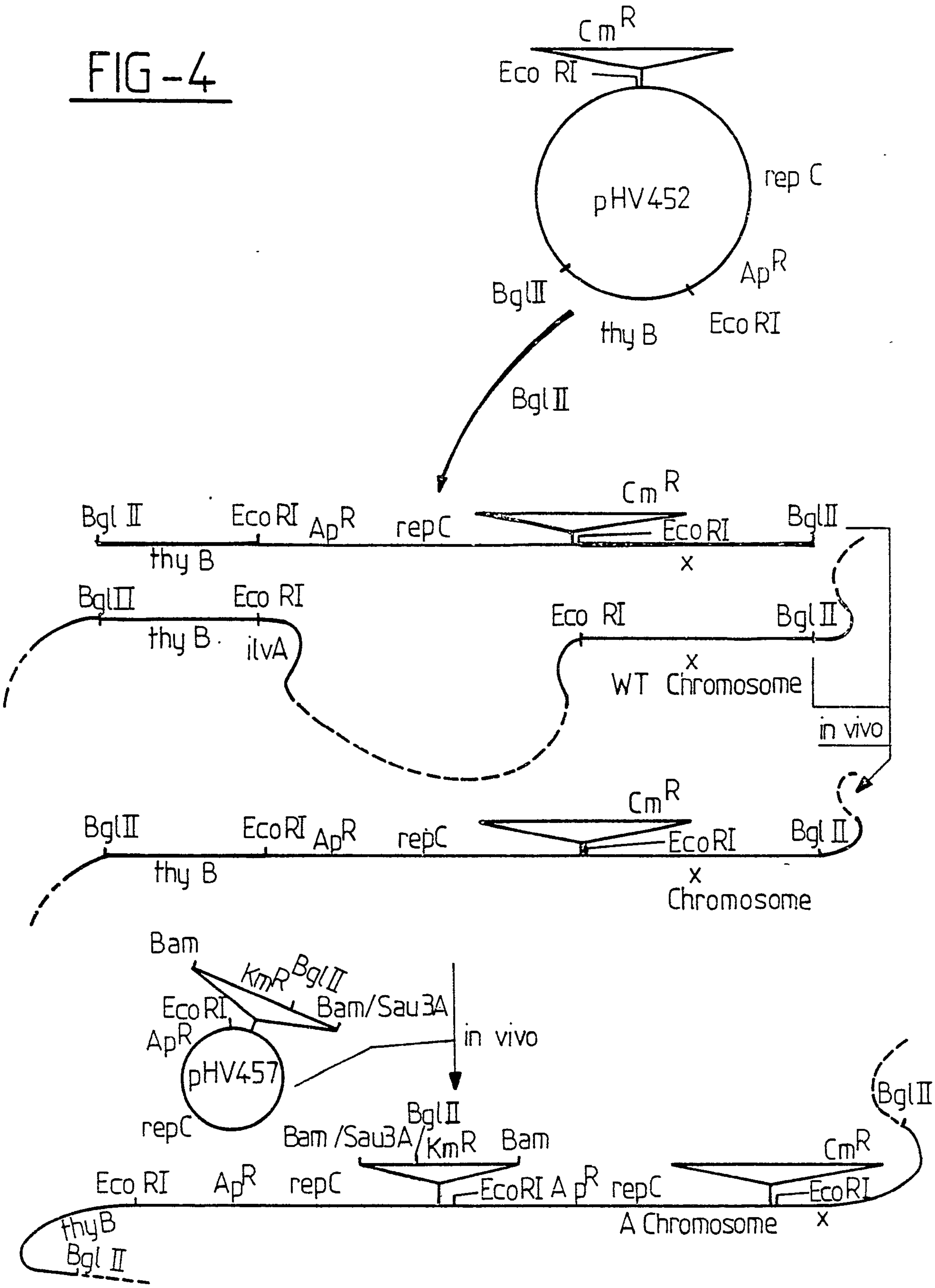
CmR
Eco RI
pHV452
rep C
ApR
BglII
thy B
Eco RI
Bgl II
Bgl II
Eco RI
ApR
repC
CmR
EcoRI
BglII
thy B
x
BglII
Eco RI
thy B
ilvA
Eco RI
Bgl II
x
WT Chromosome
in vivo
BglII
EcoRI
ApR
repC
CmR
EcoRI
Bgl II
thy B
x
Chromosome
Bam
KmR
BglII
EcoRI
Bam/Sau3A
ApR
pHV457
repC
in vivo
BglII
Bam /Sau3A
KmR
Bam
Eco RI
ApR
repC
EcoRI
ApR
repC
CmR
EcoRI
BglII
thyB
Bgl II
A Chromosome
x

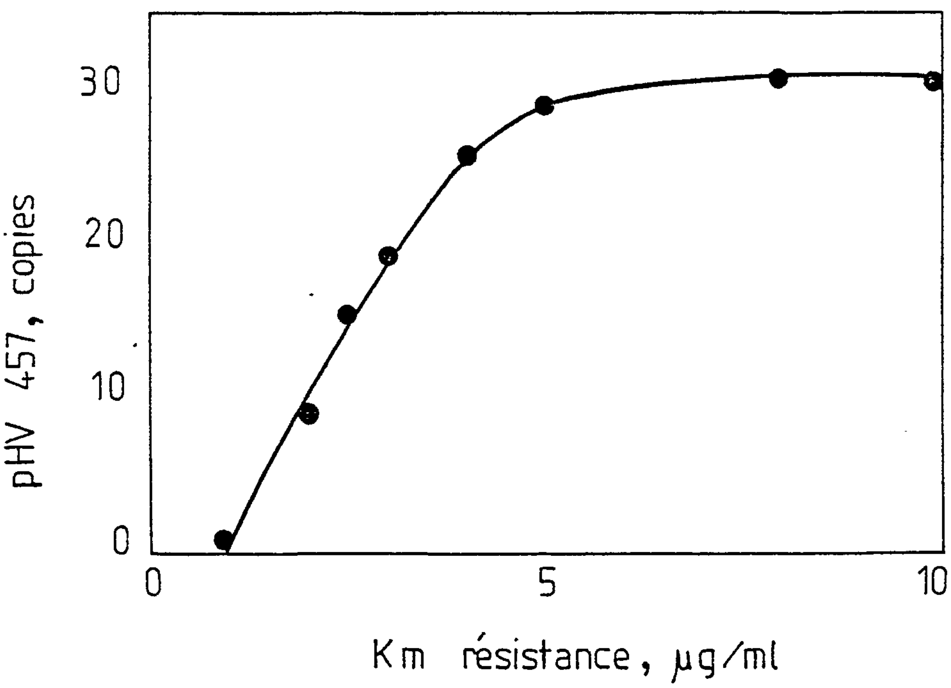
30
20
10
0
pHV 457, copies
0
5
10
Km résistance, µg/ml

FIG-6

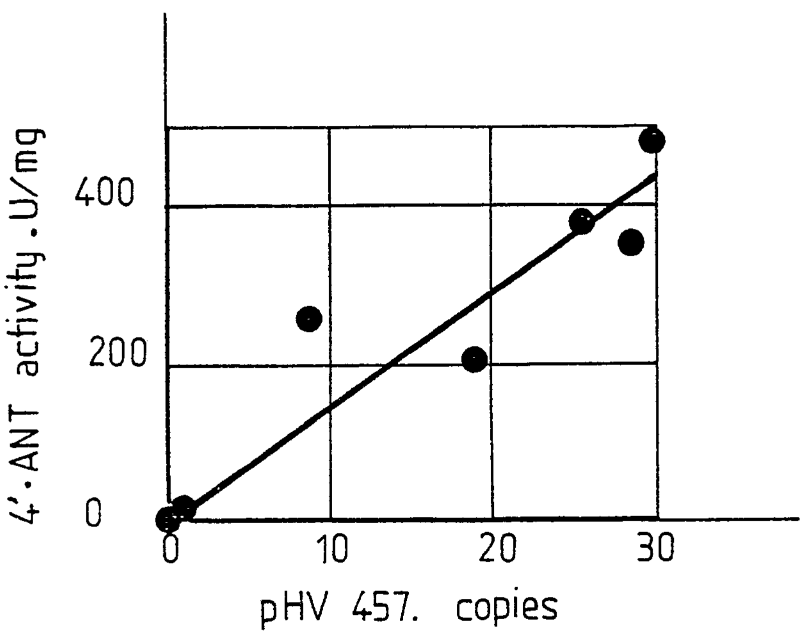
400
200
0
4'-ANT activity . U/mg
0
10
20
30
pHV 457. copies

FIG-7

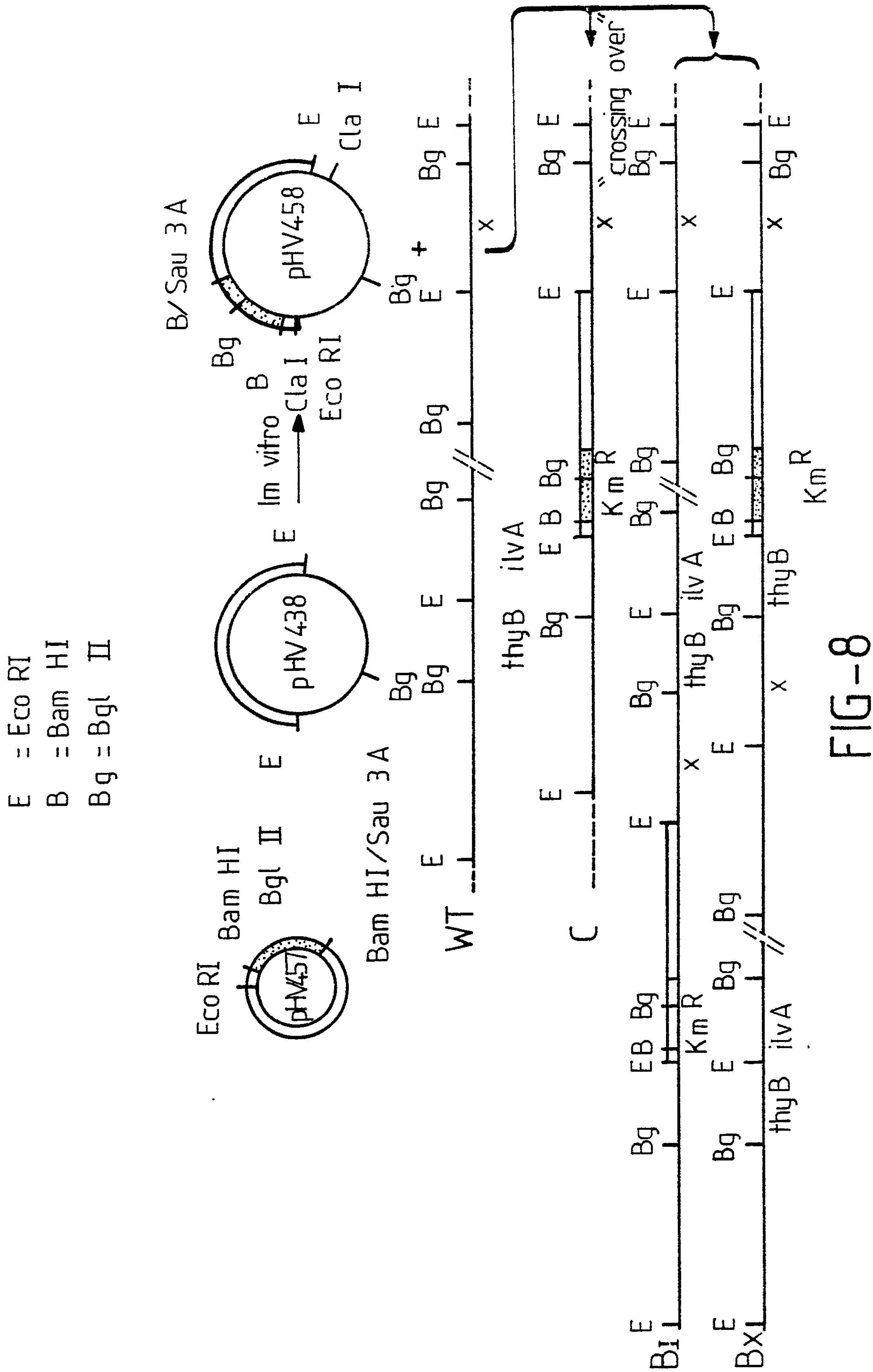
E = Eco RI
B = Bam HI
Bg = Bgl II
Eco RI
Bam HI
Bgl II
pHV457
Bam HI/Sau 3A
pHV438
E
Bg
Im vitro
Cla I
Eco RI
B
Bg
B/Sau 3A
pHV458
Cla I
E
WT
C
BI
BX
thyB
ilvA
Km R
"crossing over"

FIG-8

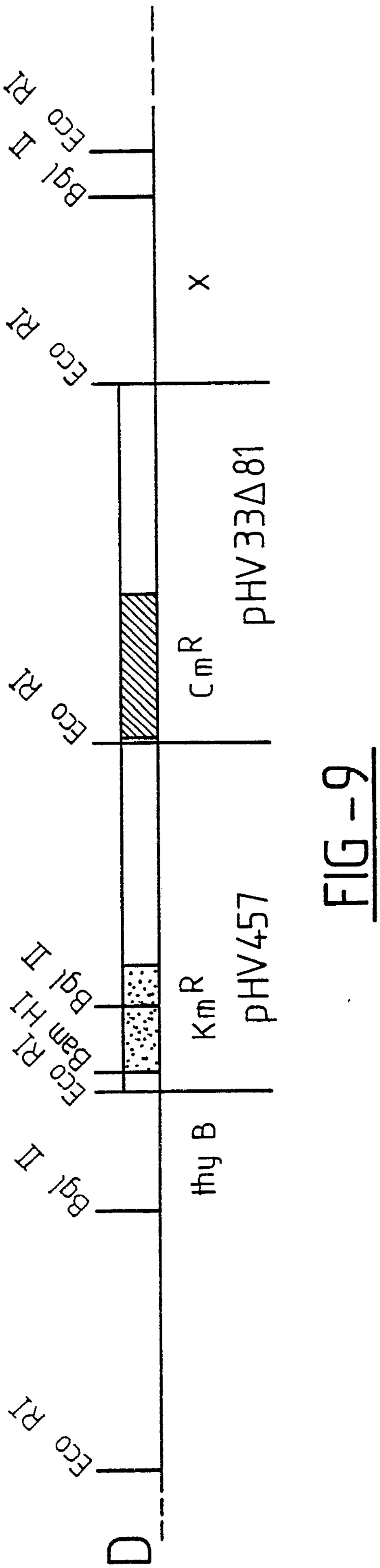
D
Eco RI
Bgl II
thy B
Eco RI
Bam HI
Bgl II
KmR
pHV457
Eco RI
CmR
pHV33Δ81
Eco RI
x
Bgl II
Eco RI

FIG-9

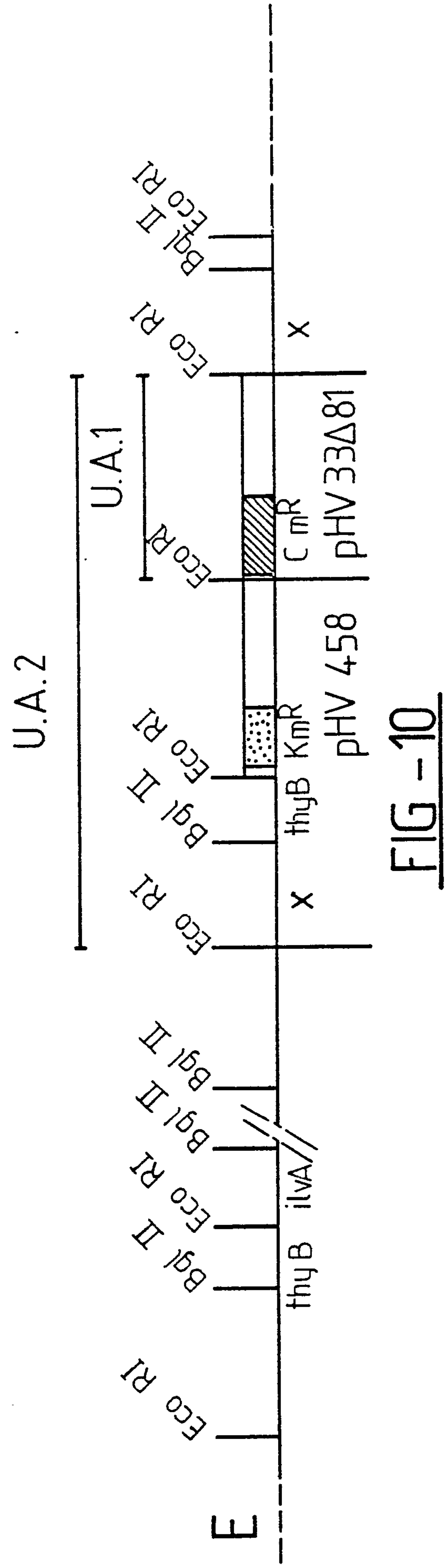
E
Eco RI
Bgl II
thy B
Eco RI
ilvA
Bgl II
Bgl II
Eco RI
x
Bgl II
thyB
Eco RI
KmR
pHV 458
Eco RI
CmR
pHV33Δ81
U.A.2
U.A.1
Eco RI
x
Bgl II
Eco RI

FIG-10